# FASCICULE DE BREVET EUROPEEN

(11) **EP 0 378 940 B1**
(45) Date de publication et mention de la délivrance du brevet: **30.03.1994**
(21) Numéro de dépôt: 89403513.8
(22) Date de dépôt: 15.12.1989
(51) Int. Cl.: H01B 7/28, H01B 3/48

(54) **Matériau composite apte à gonfler en présence d'eau, supports utilisables pour sa fabrication et ses utilisations**
Quellfähiger Verbundstoff, geeigenete Substrate zu seiner Herstellung und seine Anwendungen
Water-swellable composite material substrates useful for its manufacture and its uses

(30) Priorité: 20.12.1988 FR 8816837
(43) Date de publication de la demande: 25.07.1990
(73) Titulaire: INTISSEL, F-59393 Wattrelos (FR)
(72) Inventeur: Bottiglione, Vincent, F-59510 Hem (FR); Mutschler, Gérard, F-59110 La Madeleine (FR)
(74) Mandataire: Behaghel, Pierre

(56) Documents cités:
- EP-A- 0 024 631
- EP-A- 0 188 959
- WO-A-80/00895

## Description

L'invention concerne un matériau composite apte à gonfler en présence d'eau ou d'une solution aqueuse.

Plus précisément, l'invention a pour objet un matériau composite de type "sandwich", comprenant essentiellement un mélange de poudre hydroexpansible et de poudre thermocollante fixant l'une sur l'autre deux couches de matériaux identiques ou différents, dont l'un au moins est au moins partiellement hydrosoluble, un matériau au moins partiellement hydrosoluble pour la fabrication de ce composite et les applications de ce composite.

On connaît déjà des matériaux composites aptes à gonfler, sans se dissoudre de façon importante, en présence d'eau. De tels matériaux sont en général utilisés dans l'industrie des câbles électriques. Ils sont alors introduits, sous forme de ruban, dans l'enveloppe du câble pour assurer son étanchéité longitudinale, en formant, par gonflement, un bouchon lors de l'entrée accidentelle d'eau dans le câble tant par ses extrémités qu'au niveau d'un percement de l'enveloppe, empêchant ainsi la propagation de l'eau à l'intérieur du câble.

Parmi les rubans de ce type, on peut citer celui qui est commercialisé par la Société Freundenberg, Weinheim, R.F.A. sous la dénomination VILEDON-FIBREX® et les références connues à ce jour K3310, K3312, K3313, K3303C et K3002C, celui qui est commercialisé par la Société LANTOR, Veenendaal, Pays-Bas sous les références connues à ce jour : 3C 115, 3C 116, 3E 110, 3E 111, 3E 113, 3E 114, 3E 115 et 3E 116, auquel il est fait référence dans le brevet FR 81 02863 et celui commercialisé par la Société GECA TAPES, BAARLE NASSEAU, PAYS-BAS, sous les références connues à ce jour : GT 100, GT 101, GT 200, GT 201, GT 1000, GT 205.

Ces rubans sont essentiellement constitués d'une poudre de polymère hydroexpansible prise "en sandwich" entre deux couches de nontissé conventionnel.

La cohésion du composite est obtenue par incorporation à la poudre de polymère hydroexpansible, d'une substance (poudre, fibre ou autre) thermoplastique. Sous l'action conjuguée de la pression et de la chaleur, la substance thermoplastique se ramollit, devient collante, et assure ainsi la cohésion du composite.

Lors de cette action de collage, la nature des nontissés et leur association avec la substance thermocollante provoquent un effet défavorable : en effet, les deux couches de nontissés sont liées fortement et durablement par la masse fondue de poudre thermoplastique. Ceci a pour effet de limiter le gonflement du polymère hydroexpansible en présence d'eau ou d'une solution aqueuse.

Selon l'invention, on a maintenant trouvé que l'on pouvait améliorer considérablement les propriétés d'un tel matériau composite en remplaçant l'une au moins des deux couches de nontissés conventionnels par un ou deux support(s) solide(s), plat(s) partiellement ou totalement soluble(s) dans l'eau ou les solutions aqueuses (ci-après pour simplifier : l'eau).

L'invention a entre autres pour objet le matériau composite ainsi obtenu.

L'invention a plus particulièrement pour objet un matériau composite comprenant essentiellement un mélange de poudre hydroexpansible et de poudre thermocollante, pris en sandwich entre deux supports solides, plats dont au moins l'un peut se dissoudre façon importante en présence d'eau.

Selon un mode préféré de réalisation, ledit mélange est constitué de :
- 80 à 60 % en poids de poudre hydroexpansible ; et
- 20 à 40 % en poids de foudre thermocollante.

Un tel matériau permet de maintenir l'eau à l'endroit où elle est introduite et présente donc de nombreuses applications.

Ainsi, il peut être utilisé comme ruban d'étanchéité dans des câbles, notamment électriques ou de télécommunications, mais également comme activateur de germination ou pour la transplantation des jeunes pousses, en médecine ou chirurgie comme "éponge", ou dans le domaine de l'hygiène, par exemple dans des couches jetables.

Les supports et en particulier le ou les support(s) au moins partiellement hydrosoluble(s) de ce matériau sont choisis essentiellement en fonction de l'application envisagée.

Ils peuvent être constitués notamment d'un nontissé, d'un textile, d'un papier ou d'un film synthétique. Pour la plupart des applications, notamment dans l'industrie des câbles de transport d'énergie et de télécommunications, ils sont de préférence souples.

Le polymère hydroexpansible peut être constitué de tout polymère ou mélange de polymères compatible avec le support et l'application envisagés et capable de gonfler de façon importante en présence d'eau ou de solutions aqueuses par "emmagasinage" de celles-ci, tout en y étant insoluble, c'est-à-dire en présentant une solubilité dans ces liquides inférieure à 5 %.

Avantageusement, le polymère hydroexpansible est choisi de telle sorte qu'il soit capable d'absorber au moins 15 fois son propre poids d'eau.

Le polymère hydroexpansible peut être choisi notamment parmi les polymères acryliques modifiés, les amidons greffés, les polyacrylamides, la carboxyméthylcellulose et ses dérivés et avantageusement de façon générale parmi les polymères répondant à la définition des "superabsorbants", c'est-à-dire les polymères "insolubles" (au sens indiqué plus haut) dans les fluides qu'ils absorbent et absorbant au moins 15 fois leur propre poids d'eau ou de solution aqueuse.

En tant que polymères superabsorbants, on peut citer à titre indicatif mais en aucune façon limitatif:
- le polymère acrylique commercialisé par la société JAPAN CATALYTIC CHEMICAL sous la dénomination AQUALIC®;
- le polymère d'acrylamide commercialisé par la société CYANAMID sous la dénomination HYDROBLOCK®;
- le polymère de carboxyméthylcellulose commercialisé par la société HERCULES sous la dénomination AQUASORB®;
- le polymère amidon/acide polyacrylique obtenu par greffage et commercialisé par la société LION sous la dénomination LION-POLYMER®; et
- le polymère polyacrylate de sodium commercialisé par la société GRAIN PROCESSING sous la dénomination WATER-LOCK J®.

La poudre thermocollante est choisie parmi les polymères thermoplastiques capables de coller deux surfaces fibreuses ou pleines et compatibles avec les autres matériaux utilisés et l'usage envisagé.

Parmi les poudres thermocollantes de polymères que l'on peut avantageusement utiliser dans le cadre de l'invention on peut citer, à titre d'exemples non limitatifs :
- les polyéthylènes commercialisés par la Société CDF CHIMIE sous la marque LOTRENE® ;
- les polyéthylènes commercialisés par la Société EXXON sous la marque ESCORENE® ; et
- les polyamides commercialisés par la Société EMS sous la marque GRILTEX®.

Pour l'utilisation, en tant qu'agent d'étanchéité dans l'industrie des câbles électriques pour le transport de l'énergie moyenne et haute tension, il est avantageux de rendre le matériau composite conducteur de l'électricité, ceci afin d'obtenir une conductibilité acceptable dans son épaisseur et en surface.

Pour ce faire, au moins l'un des deux supports solides plats, est chargé au préalable d'un matériau permettant d'obtenir une résistance surfacique et transversale adaptée au type de câble fabriqué. On introduit en général à cet effet de 5 à 20 g/m² de noir de carbone conducteur dans ledit support ou chacun desdits supports lors de sa (leur) fabrication.

Du noir de carbone conducteur peut également être introduit dans le mélange de poudre hydroexpansible et de poudre thermocollante dans des proportions telles qu'on obtienne une répartition de 8 à 20 g/m² de noir de carbone dans le mélange pris en sandwich.

Selon un mode préféré de réalisation de l'invention, on dispose ainsi d'un matériau composite comprenant essentiellement un mélange de 80 à 60 % en poids de poudre hydroexpansible et de 20 à 40 % en poids de poudre thermocollante, pris en sandwich entre deux supports solides, plats, dont l'un au moins est au moins partiellement hydrosoluble, et de 5 à 60 g/m² de noir de carbone conducteur.

Le matériau composite, selon l'invention, peut être fabriqué de façon connue par saupoudrage d'un support solide, plat avec le mélange de poudre hydroexpansible et de poudre thermocollante, contenant le cas échéant du noir de carbone conducteur. Cet ensemble est ensuite chauffé à une température suffisante pour faire fondre la poudre thermocollante. Ce chauffage peut être effectué dans un séchoir, un four, par exemple à air chaud pulsé, ou sous des rampes de chauffage à infra-rouges. Le deuxième support solide, plat, est ensuite appliqué et pressé sur l'ensemble chauffé, au moyen de cylindres.

Cet assemblage peut aussi être réalise directement au moyen de deux cylindres chauffés (calandre).

Selon un mode avantageux de réalisation, au moins l'un des supports du composite selon l'invention est constitué d'un nontissé partiellement hydrosoluble. Les nontissés de ce type, partiellement hydrosolubles, utilisables pour la fabrication du matériau composite selon l'invention, comprennent essentiellement en poids :
- 95 à 55 % de fibres non hydrosolubles ;
- 5 à 35 % de liant hydrosoluble ; et
- 0 à 10 % d'agent tensio-actif.

De tels nontissés peuvent en outre, notamment pour l'utilisation dans les câbles électriques, comprendre du noir de carbone conducteur.

Ainsi, selon un mode de réalisation avantageux, les nontissés partiellement hydrosolubles, comprennent essentiellement en poids :
- 76 à 28 % de fibres non hydrosolubles ;
- 4 à 18 % de liant hydrosoluble ;
- 0 à 4 % d'agent tensio-actif ; et
- 20 à 50 % de noir de carbone conducteur.

Les fibres non hydrosolubles peuvent être naturelles, artificielles ou synthétiques. Il peut s'agir notamment de fibres de viscose, de fibres de polyester, de fibres acryliques ou de mélanges de telles fibres.

Comme exemples de telles fibres on peut citer, à titre purement illustratif :
- les fibres de polyester, commercialisées par la Société EMS, sous la dénomination commerciale GRILENE® ;
- les fibres de polyester, commercialisées par la Société DU PONT DE NEMOURS, sous la dénomination commerciale DACRON® ;
- les fibres de polyamide, commercialisées par la Société EMS sous la dénomination GRILON® ;
- les fibres de polypropylène, commercialisées par la Société STEEN sous la dénomination POLYSTEEN® ; et
- les fibres de viscose, commercialisées par la Société LENZING.

Le liant hydrosoluble est choisi parmi les liants hydrosolubles capables de fixer des masses fibreuses plates, sans modifier sensiblement leurs propriétés physico-chimiques.

Parmi les liants de ce type que l'on peut avantageusement utiliser dans le cadre de l'invention, on peut citer à titre d'exemples non limitatifs :
- les polyvinylpyrrolidones commercialisées par la société GAF, sous les dénominations commerciales PVP K 30® et PVP K 90® ;
- les polyvinylpyrrolidones et copolymères de vinylpyrrolidone-acétate de vinyle commercialisés par la société BASF (RFA) sous les dénominations commerciales de LUVISKOL K30-K90® ; et
- les hydroxypropylcelluloses commercialisées par la société HERCULES (E.U.A.) sous les dénominations commerciales KLUCEL H, M, G, J, L et E®.

Bien que la présence d'un agent tensio-actif ne soit pas indispensable, il a été établi qu'elle joue un rôle favorable, notamment dans la répartition du liant lors de la fabrication du nontissé.

On utilise de préférence, en tant qu'agent tensio-actif, un agent tension tensio-actif anionique ou non ionique.

Parmi les agents tensio-actifs qui peuvent être utilisés dans le cadre de l'invention, on peut citer, à titre d'exemples non limitatifs :
- le produit commercialisé par la société ROHM AND HAAS (E.U.A.) sous les dénominations commerciales de TRITON X 100® et TRITON GR 5 M® ;
- le produit commercialisé par la société BYK-MALLINCKRODT sous la dénomination ANTI-TERRA-U® ;
- le produit commercialisé par la société PETROCHEMICALS CO., INC. sous la dénomination de MORWET EFN®;
- les produits commercialisés par la société AIR PRODUCTS sous la dénomination de SURFYNOL® ; et
- le produit commercialisé par la société BASF (R.F.A.) sous la dénomination de LEOPHEN RA®.

La fabrication de ce nontissé partiellement hydrosoluble peut être réalisée, par exemple de façon classique, par le procédé appelé "voie sèche". Ce procédé consiste en une opération de cardage des fibres, destinée à rendre ces fibres parallèles entre elles et à produire un voile homogène. Ce voile est ensuite fixé au moyen du liant hydrosoluble, utilisé par exemple sous forme de solution en phase aqueuse dont la concentration est dosée de façon à obtenir un nontissé suffisamment solide pour l'application envisagée, tout en permettant une dissolution la plus rapide possible lorsque le complexe devra remplir son rôle au contact de l'eau ou d'une solution aqueuse.

Cette concentration dépend notamment de la nature et des caractéristiques physiques des fibres à lier, d'une part, et du liant utilisé, d'autre part. Dans la plupart des cas, elle peut être de 3 à 20 %, avantageusement de 7 %. La solution de liant hydrosoluble est déposée sur le voile, par exemple sous forme d'une mousse réalisée au moyen d'un dispositif connu sous le nom de "mousseur".

Les additifs éventuels (tels que agent tensio-actif, noir de carbone conducteur) peuvent être incorporés à la solution avant ou pendant le moussage.

Le voile ainsi imprégné est ensuite séché, par exemple par un séchoir rotatif à air traversant, par un système de cylindres chauffés, appelé maniques, par un tunnel chauffant ou par tout autre dispositif convenant a cette application.

Le nontissé peut ensuite, soit être enroulé pour être utilisé ultérieurement, soit entrer immédiatement dans le processus de fabrication du composite selon l'invention.

Selon un autre mode avantageux de réalisation, au moins l'un des supports du composite selon l'invention est constitué d'un nontissé au moins partiellement hydrosoluble formé, en tout ou partie, de fibres au moins partiellement hydrosolubles et thermocollantes, la liaison des fibres étant alors réalisée par liage thermique.

Dans ce contexte, a titre d'exemple, le nontissé peut être constitué de :
- 50 à 100 % de fibres de PVA (alcool polyvinylique) ; et
- 50 à 0 % de fibres naturelles, artificielles ou synthétiques, telles que par exemple celles mentionnées précédemment à titre d'exemples.

En tant que fibres de PVA, on peut citer celles commercialisées par Société KURARAY sous la marque KURALON®.

Selon encore un autre mode de réalisation, le composite selon l'invention peut comprendre, en tant que support, au moins un textile, au moins partiellement hydrosoluble qui est tissé ou tricoté.

En tant que textile nouveau de ce type on peut citer un textile tissé ou tricoté, au moins partiellement hydrosoluble, qui est essentiellement constitué de :
- 50 à 100 % de fils PVA (alcool polyvinylique),
- 50 à 0 % de fils conventionnels naturels, artificiels ou synthétiques, obtenus par exemple à partir des fibres mentionnées précédemment à titre d'exemples.

Le papier, au moins partiellement hydrosoluble que l'on peut également utiliser comme support dans le composite selon l'invention, peut être réalisé de façon classique par dispersion de fibres naturelles, artificielles ou synthétiques dans un bain à forte concentration en liant hydrosoluble.

En tant que papier de ce type, on peut citer le papier commercialisé par la Société NEDI (FRANCE), sous la dénomination de "Papier hydro dispersable".

En tant que film support hydrosoluble on peut utiliser un film synthétique d'alcool polyvinylique. Dans ce contexte, on peut citer les films commercialisés par la Société NEDI (FRANCE), sous la dénomination de NEDOL®.

Comme il a déjà été indiqué plus haut, le matériau composite selon l'invention peut avoir des applications variées.

Ainsi il peut être utilisé en tant qu'absorbant de l'eau ou des liquides physiologiques, notamment en médecine et en chirurgie ou dans le domaine des produits sanitaires tels que couches à jeter etc.

Il peut en particulier être incorporé dans la fabrication des couches pour bébés, sous forme de bandes ou de pièces découpées, permettant ainsi de renforcer l'absorption et la rétention d'urine, notamment à des endroits particuliers dépendant de la morphologie des bébés.

Le composite selon l'invention peut également être utilisé en agriculture comme activateur de germination pour maintenir l'eau à l'endroit où la graine est déposée ; cette localisation précise est particulièrement intéressante lorsque l'eau est enrichie en substances favorisant la croissance, telles que des substances nutritives. De plus, des substances telles que des composés anticryptogamiques, fongicides, engrais peuvent facilement être incorporés dans le matériau composite au moment du poudrage.

Le matériau composite selon l'invention peut également être utilisé pour la transplantation des jeunes pousses. Celles-ci sont emballées avec leur motte dans le matériau composite et peuvent subir, après trempage dans de l'eau, une transplantation sans risque.

Comme indiqué plus haut, une autre application importante du matériau composite selon l'invention est son utilisation dans les câbles pour assurer leur étanchéité longitudinale.

En cas de contact avec de l'eau, l'un au moins des deux supports se décompose, au moins partiellement, découvrant les sites hydrophiles du polymère hydroexpansible. Il en résulte un gonflement compact et rapide. Dans le même temps, le polymère hydroexpansible est libéré par la décomposition du ou des supports. Cette libération permet un déplacement du gel qui peut s'orienter vers les zones de passage préférentiel de l'eau, assurant ainsi une excellente efficacité in situ.

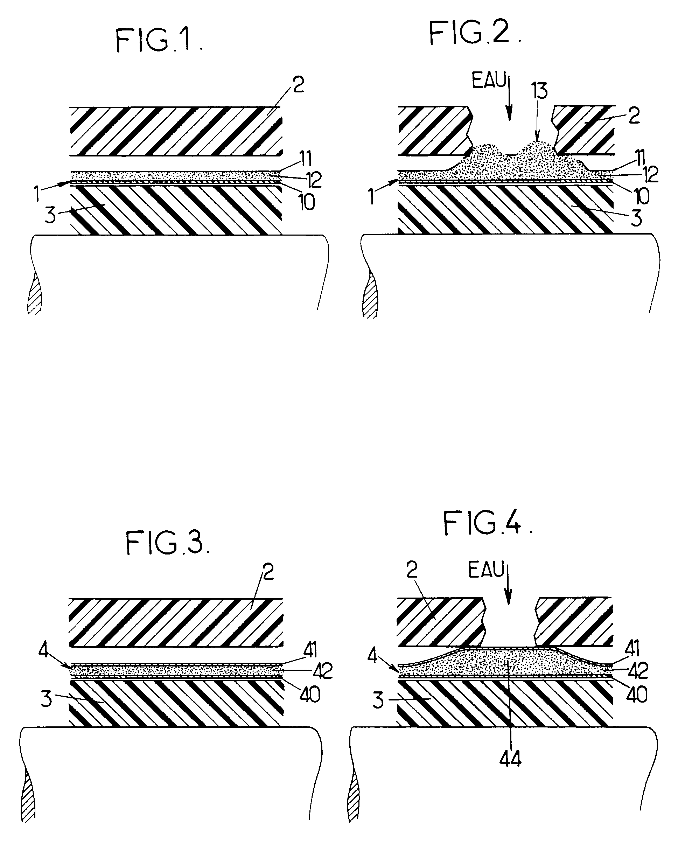

En se reportant aux figures 1 et 2 de la planche de dessins annexée, il est montré ci-après comment se comporte le matériau composite selon l'invention lorsqu'il est incorporé dans la gaine d'un câble, en présence d'eau traversant accidentellement l'enveloppe externe du câble.

Le matériau composite, selon l'invention 1, constitué de deux supports solides, plats 10 et 11 dont l'un au moins 11 est totalement ou partiellement hydrosoluble, liés par un mélange 12 de poudre de polymère hydroexpansible et de poudre de polymère thermocollant, est placé entre l'enveloppe externe 2 et l'âme, ou plus généralement l'enveloppe interne 3, du câble. De par la construction du câble, ses parties 2 et 3 sont souvent séparées par un léger vide. De plus, elles s'écartent l'une de l'autre de façon plus ou moins importante au cours de l'utilisation du câble (figure 1).

Si de l'eau pénètre accidentellement dans le câble (figure 2), à travers l'enveloppe 2 externe et traverse le support solide, plat, au moins partiellement hydrosoluble 11 du matériau composite 1, elle dissout au moins partiellement, quasi instantanément, ce support plat et solide et entre en contact avec la poudre de polymère hydroexpansible, au voisinage de l'endroit de sa pénétration dans le câble.

Le polymère hydroexpansible qui était maintenu entre les deux supports est alors libéré à cet endroit et a ainsi la possibilité de se déplacer, tout en gélifiant, pour former autour de la zone de pénétration de l'eau un bourrelet 13 qui empêche la propagation de l'eau dans le câble.

Le polymère hydroexpansible qui n'est plus prisonnier des supports peut développer toute sa capacité de gonflement pour bloquer l'entrée accidentelle d'eau dans le câble. Cette capacité et cette rapidité sont améliorées par rapport à l'art antérieur où la force d'hydroexpansion devait d'abord servir à décoller les deux supports non hydrosolubles, entre lesquels le gel demeurait prisonnier un laps de temps important.

De plus, avec le composite selon l'invention 1, le gel créé est totalement efficace car il est libre et s'accumule à proximité de la zone d' infiltration.

Il va de soi que cette description n'est donnée qu'à titre purement illustratif et qu'en particulier on pourrait prévoir d'autres dispositions à l'intérieur du câble sans sortir du cadre de l'invention.

Les figures 3 et 4 montrent ce qui se passe dans le cas de l'utilisation des composites de l'art antérieur tel que défini plus haut.

Le matériau composite 4 est constitué d'une couche de mélange 42 d'un polymère hydroexpansible et d'une substance thermoplastique, prise en "sandwich" entre deux couches de nontissé non hydrosoluble 40 et 41. Lorsque de l'eau pénètre accidentellement dans le câble, le polymère hydroexpansible gonfle au niveau de la pénétration de l'eau, sans se détacher des supports : il donne une "boursouflure" 43 juste à ce niveau, mais ne peut former un bourrelet autour de cette zone.

La protection conférée est donc moins efficace que selon l'invention et, comme ce polymère ne peut développer toute sa capacité de gonflement, notamment en raison de la liaison forte et durable entre les deux supports non hydrosolubles due au polymère thermoplastique, il est nécessaire d'en utiliser une plus grande quantité.

Les exemples suivants sont destinés à illustrer et mieux expliquer l'invention sans toutefois en limiter la portée.

### EXEMPLE 1

### Nontissé partiellement hydrosoluble à base de fibres classiques non hydrosolubles.

| | |
|---|---|
| - Fibres de polyester GRILENE® HTB de la société EMS (Suisse) | 73 % en poids |
| - Liant polyvinylpyrrolidone PVP K30® de la société GAF (E.U.A.) | 25 % en poids |
| - Agent mouillant TRITON GR 5 M® de la société ROHM AND HAAS (E.U.A.) | 2 % en poids |

### EXEMPLE 2

### Fabrication d'un nontissé partiellement hydrosoluble comprenant des fibres hydrosolubles thermocollantes.

| | |
|---|---|
| - Fibres de PVA KURALON® de la société KURARAY (Japon) | 70 % en poids |
| - Fibres de polyester GRILENE HTB® de la société EMS (Suisse) | 30 % en poids |

Le voile de fibres est humidifié puis lié thermiquement par calandrage.

### EXEMPLE 3

### Fabrication d'un matériau composite selon l'invention.

| COMPOSANTS | POIDS |
|---|---|
| Support non hydrosoluble, nontissé Réf. 42040 d'INTISSEL (FRANCE) | 40 g/m² |
| Poudre hydroexpansible Réf. 10 SHP de NORSOLOR (FRANCE) | 35 g/m² |
| Poudre thermoplastique de polyéthylène ESCORENE®, Réf. MP 654 de EXXON (E.U.A) | 10 g/m² |
| Support nontissé, au moins partiellement hydrosoluble, selon l'invention (en particulier selon les compositions décrites précédemment, et notamment dans les exemples 1 et 2) | 15 g/m² |

Le support non hydrosoluble est un nontissé conventionnel, conducteur ou non, fabriqué selon le procédé dit par "voie sèche", à partir de fibres de polyester et d'un liant butadiène-acrylonitrile, chargé ou non de noir de carbone conducteur. Ces fibres sont orientées de préférence parallèlement.

Sur ce support nontissé, non hydrosoluble, est ensuite déposé le mélange de poudre hydroexpansible et de poudre thermocollante au moyen d'une poudreuse constituée d'un bac de stockage de poudre, au fond duquel se situe un cylindre gravé qui délivre la poudre et dont la vitesse de rotation détermine le poids de poudre déposé. Il est dans cet exemple de 45 g/m².

Le support nontissé, non hydrosoluble, poudré passe ensuite dans un four comportant des rampes à rayonnement infra-rouge, réglé à une température suffisante pour faire fondre la poudre thermocollante.

En sortie de ce four, on déroule le support nontissé au moins partiellement hydrosoluble, conducteur ou non, que l'on applique avec pression sur le premier support poudré au moyen de deux cylindres dont on peut régler la force de pression.

## Revendications

1. Matériau composite (1) comprenant essentiellement un mélange (12) de poudre hydroexpansible et de poudre thermocollante pris en sandwich entre deux supports solides (10,11) plats, caractérisé par le fait qu'au moins l'un des supports peut se dissoudre de façon importante en présence d'eau.

2. Matériau composite selon la revendication 1, caractérisé en ce que le mélange est constitué de :
- 80 à 60 % en poids de poudre hydroexpansible ; et
- 20 à 40 % en poids de poudre thermocollante.

3. Matériau composite selon l'une des revendications 1 ou 2, caractérisé en ce que les supports solides, sont constitués d'un nontissé, d'un textile, d'un papier ou d'un film synthétique.

4. Matériau composite selon l'une quelconque des revendications 1 à 3, caractérisé en ce que les supports solides sont souples.

5. Matériau composite selon l'une quelconque des revendications 1 à 4, caractérisé en ce que le polymère hydroexpansible est un superabsorbant.

6. Matériau composite selon l'une quelconque des revendications 1 à 5, caractérisé en ce qu'il comprend de 5 à 60 g/m² de noir de carbone conducteur.

7. Matériau composite selon la revendication 6, caractérisé en qu'il comprend de 5 à 20 g/m² de noir de carbone conducteur dans au moins l'un des supports.

8. Matériau composite selon l'une ou l'autre des revendications 6 et 7, caractérisé en ce qu'il comprend de 8 à 20 g/m² de noir de carbone conducteur dans le mélange pris en sandwich.

9. Matériau composite selon l'une quelconque des revendications 1 à 8, caractérisé en ce que au moins l'un des supports solides est constitué d'un nontissé partiellement hydrosoluble comprenant essentiellement en poids :
- 95 à 55 % de fibres non hydrosolubles ;
- 5 à 35 % de liant hydrosoluble ; et
- 0 à 10 % d'agent tensio-actif.

10. Matériau composite selon l'une des revendications 6, 7 ou 8 caractérisé en ce qu'au moins l'un des supports solides est constitué d'un nontissé partiellement hydrosoluble comprenant essentiellement en poids :
- 76 à 28 % de fibres non hydrosolubles ;
- 4 à 18 % de liant hydrosoluble ;
- 0 à 4 % d'agent tensio-actif ; et
- 20 à 50 % de noir de carbone conducteur.

11. Matériau composite selon l'une quelconque des revendications 1 à 8 caractérisé en ce qu'au moins l'un des supports solides est un nontissé partiellement ou totalement hydrosoluble constitué, en tout ou partie, de libres hydrosolubles et thermocollantes et dans lequel la liaison des fibres est assurée par liage thermique.

12. Matériau composite selon l'une quelconque des revendications 1 à 8, caractérisé en que l'un au moins des supports solides est tissé ou tricoté.

13. Utilisation du matériau composite selon l'une des revendications 1 à 12 comme agent d'étanchéité dans les câbles.

14. Utilisation du matériau composite selon l'une des revendications 1 à 5, 9, 11 et 12 en agriculture.

15. Utilisation du matériau composite selon l'une des revendications 1 à 5, 9, 11 et 12 en médecine ou en chirurgie.

16. Utilisation du matériau composite selon l'une des revendications 1 à 5, 9, 11 et 12 dans le domaine de l'hygiène.

## Claims

1. Composite material (1) comprising essentially a mixture (12) of hydroexpandable powder and thermobonding powder sandwiched between two flat solid supports (10,11), characterized by the fact that at least one of the supports can substantially dissolve in the presence of water.

2. Composite material according to claim 1, characterized in that the mixture is constituted of :
- 80 to 60% by weight of hydroexpandable powder ; and
- 20 to 40% by weight of thermobonding powder.

3. Composite material according to anyone of the claims 1 or 2, characterized in that the solid supports are consisting of a non-woven fabric, a textile, a paper or a synthetic film.

4. Composite material according to anyone of the claims 1 to 3, characterized in that the solid supports are flexible.

5. Composite material according to anyone of the claims 1 to 4, characterized in that the hydroexpandable polymer is a superabsorbent.

6. Composite material according to anyone of the claims 1 to 5, characterized in that it contains from 5 to 60g/m² of conducting carbon black.

7. Composite material according to claim 6, characterized in that it contains from 5 to 20g/m² of conducting carbon black in at least one of the supports.

8. Composite material according to anyone of the claims 6 and 7, characterized in that it contains from 8 to 20g/m² of conducting carbon black in the sandwiched mixture.

9. Composite material according to anyone of the claims 1 to 8, characterized in that at least one of the solid supports is constituted of a non-woven fabric partially hydrosoluble comprising essentially by weight :
- 95 to 55% of non hydrosoluble fibers ;
- 5 to 35% of hydrosoluble binder ; and
- 0 to 10% of surface-active agent.

10. Composite material according to anyone of the claims 6, 7 or 8, characterized in that at least one of the solid supports is constituted of a non-woven fabric partially hydrosoluble comprising essentially by weight :
- 76 to 28% of non hydrosoluble fibers ;
- 4 to 18% of hydrosoluble binder ;
- 0 to 4% of surface-active agent ; and
- 20 to 50% of conducting carbon black.

11. Composite material according to anyone of the claims 1 to 8, characterized in that at least one of the solid supports is a non-woven fabric partially or completely hydrosoluble constituted, wholly or partially, of hydrosoluble and thermobonding fibers and wherein the bonding of the fibers is provided by heat bonding.

12. Composite material according to anyone of the claims 1 to 8, characterized in that at least one of the solid supports is woven or knitted.

13. Use of the composite material according to anyone of the claims 1 to 12 as waterproofness agent in cables.

14. Use of the composite material according to anyone of the claims 1 to 5, 9, 11 and 12 in agriculture.

15. Use of the composite material according to anyone of the claims 1 to 5, 9, 11 and 12 in medicine or in surgery.

16. Use of the composite material according to anyone of the claims 1 to 5, 9, 11 and 12 in the hygiene field.

## Patentansprüche

1. Verbundmaterial (1) umfassend im wesentlichen ein zwischen zwei flachen festen Trägern (10,11) sandwichartig angeordnetes Gemisch (12) aus durch Wasser ausdehnbarem Pulver und aus thermoklebendem Pulver, dadurch gekennzeichnet, daß wenigstens einer der Träger bei Vorhandensein von Wasser in beträchtlichem Maße auflösbar ist.

2. Verbundmaterial nach Anspruch 1, dadurch gekennzeichnet, daß das Gemisch gebildet ist aus:
- 80 bis 60 Gew.-% durch Wassser ausdehnbarem Pulver; und
- 20 bis 40 Gew.-% thermoklebendem Pulver.

3. Verbundmaterial nach Anspruch 1 oder 2, dadurch gekennzeichnet, daß die festen Träger aus einem Faservlies, einem Gewebe, einem Papier oder einem synthetischen Film gebildet sind.

4. Verbundmaterial nach einem der Ansprüche 1 bis 3, dadurch gekennzeichnet, daß die festen Träger flexibel sind.

5. Verbundmaterial nach einem der Ansprüche 1 bis 4, dadurch gekennzeichnet, daß das durch Wasser ausdehnbare Polymer ein Super-Absorptionsmittel ist.

6. Verbundmaterial nach einem der Ansprüche 1 bis 5, dadurch gekennzeichnet, daß es 5 bis 60 g/m² leitendes Rußschwarz umfaßt.

7. Verbundmaterial nach Anspruch 6, dadurch gekennzeichnet, daß es in wenigstens einem der Träger 5 bis 20 g/m² leitendes Rußschwarz umfaßt.

8. Verbundmaterial nach Anspruch 6 oder 7, dadurch gekennzeichnet, daß es 8 bis 20 g/m² leitendes Rußschwarz in dem sandwichartig angeordneten Gemisch umfaßt.

9. Verbundmaterial nach einem der Ansprüche 1 bis 8, dadurch gekennzeichnet, daß wenigstens einer der festen Träger aus einem teilweise wasserlöslichen Faservlies gebildet ist, welches im wesentlichen umfaßt:
- 95 bis 55 Gew.-% wasserunlösliche Fasern;
- 5 bis 35 Gew.-% wasserlösliches Bindemittel; und
- 0 bis 10 Gew.-% grenzflächenaktiven Stoff.

10. Verbundmaterial nach einem der Ansprüche 6, 7 oder 8, dadurch gekennzeichnet, daß wenigstens einer der festen Träger aus einem teilweise wasserlöslichen Faservlies gebildet ist, welches im wesentlichen umfaßt:
- 76 bis 28 Gew.-% wasserunlösliche Fasern;
- 4 bis 18 Gew.-% wasserlösliches Bindemittel;
- 0 bis 4 Gew.-% grenzflächenaktiven Stoff; und
- 20 bis 50 Gew.-% leitendes Rußschwarz.

11. Verbundmaterial nach einem der Ansprüche 1 bis 8, dadurch gekennzeichnet, daß wenigstens einer der festen Träger ein teilweise oder völlig wasserlösliches Faservlies ist, welches im ganzen oder zum Teil aus wasserlöslichen und thermoklebenden Fasern gebildet ist und in welchem die Verbindung der Fasern durch thermische Bindung sichergestellt ist.

12. Verbundmaterial nach einem der Ansprüche 1 bis 8, dadurch gekennzeichnet, daß wenigstens einer der festen Träger gewebt oder gewirkt ist.

13. Verwendung des Verbundmaterials nach einem der Ansprüche 1 bis 12 als Dichtungsmittel in Kabeln.

14. Verwendung des Verbundmaterials nach einem der Ansprüche 1 bis 5, 9, 11 und 12 in der Landwirtschaft.

15. Verwendung des Verbundmaterials nach einem der Ansprüche 1 bis 5, 9, 11 und 12 in der Medizin oder in der Chirurgie.

16. Verwendung des Verbundmaterials nach einem der Ansprüche 1 bis 5, 9, 11 und 12 auf dem Gebiet der Hygiene.
